(19) Europäisches Patentamt
European Patent Office
Office européen des brevets

(11) EP 3 252 151 B1

(12) **EUROPEAN PATENT SPECIFICATION**

(45) Date of publication and mention of the grant of the patent:
**08.04.2020 Bulletin 2020/15**

(21) Application number: **16743499.2**

(22) Date of filing: **29.01.2016**

(51) Int Cl.:
***C12N 5/077*** *(2010.01)* ***C12N 5/071*** *(2010.01)*
***A61K 35/34*** *(2015.01)* ***C12Q 1/02*** *(2006.01)*

(86) International application number:
**PCT/JP2016/052571**

(87) International publication number:
**WO 2016/121896 (04.08.2016 Gazette 2016/31)**

(54) **METHOD FOR CULTIVATING VASCULAR SMOOTH MUSCLE CELLS**

VERFAHREN ZUR KULTIVIERUNG VON VASKULÄREN GLATTEN MUSKELZELLEN

PROCÉDÉ DE CULTURE DE CELLULES VASCULAIRES DE MUSCLES LISSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2015 JP 2015017839**
**26.03.2015 JP 2015065007**
**17.09.2015 JP 2015183940**

(43) Date of publication of application:
**06.12.2017 Bulletin 2017/49**

(73) Proprietors:
- **Nissan Chemical Corporation**
**Tokyo (JP)**
- **National University Corporation Chiba University**
**Chiba-shi**
**Chiba 263-8522 (JP)**

(72) Inventors:
- **ISHII, Itsuko**
**Chiba-shi**
**Chiba 260-0856 (JP)**
- **KANAKI, Tatsuro**
**Shiraoka-shi**
**Saitama 349-0294 (JP)**
- **KITAHARA, Masaki**
**Shiraoka-shi**
**Saitama 349-0294 (JP)**

(74) Representative: **J A Kemp LLP**
**14 South Square**
**Gray's Inn**
**London WC1R 5JJ (GB)**

(56) References cited:
**WO-A1-2014/017513 WO-A1-2014/017513**
**WO-A2-03/002044 US-A1- 2014 106 348**

- **SHINGO TAJIMA: "Modulation of Elastin Expression and Cell Proliferation inVascular Smooth Muscle Cells in vitro.", KEIO JOURNAL OF MEDICINE., vol. 45, no. 1, 1 January 1996 (1996-01-01), pages 58-62, XP055479332, JP ISSN: 0022-9717, DOI: 10.2302/kjm.45.58**
- **MASASHI UCHIDA ET AL: "Degradation of Filamin Induces Contraction of Vascular Smooth Muscle Cells in Type-I Collagen Matrix Honeycombs", CELLULAR PHYSIOLOGY AND BIOCHEMISTRY., vol. 27, no. 6, 1 January 2011 (2011-01-01), pages 669-680, XP055479298, CH ISSN: 1015-8987, DOI: 10.1159/000330076**
- **NATORI TOMOMI ET AL: "Growth arrest of vascular smooth muscle cells in suspension culture using low-acyl gellan gum", IN VITRO CELLULAR & DEVELOPMENTAL BIOLOGY. ANIMAL, SPRINGER US, NEW YORK, vol. 53, no. 3, 6 December 2016 (2016-12-06), pages 191-198, XP036187527, ISSN: 1071-2690, DOI: 10.1007/S11626-016-0098-X [retrieved on 2016-12-06]**
- **WACHI, H. ET AL.: 'Cell cycle-dependent regulation of elastin gene in cultured chick vascular smooth-muscle cells.' BIOCHEM. J. vol. 309, no. 2, 15 July 1995, pages 575 - 579, XP055470365 DOI: 10.1042/BJ3090575**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 252 151 B1

- **AKITA, M. ET AL.: 'Phenotypic modulation and elastin formation of cultured aortic smooth muscle cells grown on and within collagen gels.' ACTA ANAT. NIPPON. vol. 68, no. 6, December 1993, pages 598 - 606, XP009505070**
- **TAKASHI SUZUKI ET AL.: 'Culture of rat aortic Bulletin of Kohno Clinical Medicine Research Institute' vol. 37, December 1987, pages 101 - 104, XP009505072**

## Description

[Technical Field]

The present invention relates to a culture technique of vascular smooth muscle cells.

[Background Art]

Vascular endothelial cells and vascular smooth muscle cells constituting blood vessels (hereinafter blood vessel cells) are often used as primary cultured cells for in vitro experiments, and established cell lines are few. Different from established cells, primary cultured blood vessel cells have been reported to retain original properties of blood vessel cells in the body, whereas tend to easily undergo property conversion upon passage and cryopreservation. Particularly, when vascular smooth muscle cells are subjected to a conventional culture using a plate (monolayer culture: two-dimensional culture), the cells proliferate and show property change such as decreased contraction ability and the like. To avoid such property change, the following three methods have been developed. (1) Culture on type I collagen gel. Vascular smooth muscle cells can be cultured while arresting proliferation (non-patent document 1). (2) Culture using a three-dimensional matrix containing type I collagen as a main component (non-patent document 2). In this case, cell proliferation is arrested and the contraction ability intrinsic to vascular smooth muscle cells is recovered. (3) Culture of vascular endothelial cells generally on a gelatin coat.

However, each culture method has problem of its own. The method of (1) is not suitable for long-term culture. Therefore, the collagen gel needs to be dissolved and vascular smooth muscle cells need to be isolated on day 3 - 5 of culture. When continuously cultured without isolation, cell proliferation starts, resulting in equivalent to the conventional two-dimensional culture. Therefore, properties close to those in the body cannot be maintained. The culture of (2) using a type I collagen three-dimensional matrix requires about 1 week for the vascular smooth muscle cells to acquire properties close to those in the body. Furthermore, since preservation in a cultured state is not possible, clinical application is difficult. The culture of (3) using a gelatin coat does not vary much from the conventional two-dimensional culture method.

As discussed above, since current culture techniques of blood vessel cells cannot avoid property conversion of cells (two-dimensional culture method), and long-term culture is difficult and complicated (three-dimensional culture method), the development of an evaluation method using blood vessel cells, a method for transporting blood vessel cells, in vivo transplantation of blood vessel cells and the like are highly restricted. Furthermore, even if vascular smooth muscle cells are cultured while inhibiting cell adhesion by coating the surface of a culture vessel with a material that inhibits cell adhesion or applying a special processing, their survival rate decreases rapidly. Therefore, development of a culture method has been desired which method realizes a culture environment in which evaluation of medicaments and the like can be conveniently performed while minimizing stimulation from the culture material to blood vessel cells, convenient cell recovery, and a non-frozen environment for cell transport that does not easily cause property conversion of cells.

The present inventors have successfully developed a medium composition capable of culturing cells and tissues while keeping the suspended state, without substantially increasing the viscosity of the liquid medium (patent documents 1 and 2).

[Document List]

[Patent documents]

[patent document 1]WO 2014/017513 A1
[patent document 2]US 2014/0106348 A1

[non-patent document]

[non-patent document 1] Koyama et al., Cell 1996; 87: 1069-78
[non-patent document 2]Ishii et al., Atherosclerosis 2001; 158: 377-84

[SUMMARY OF THE INVENTION]

[Problems to be Solved by the Invention]

An object of the present invention is to provide a technique for culturing vascular smooth muscle cells for a long term without proliferation while avoiding property change.

[Means of Solving the Problems]

The present inventors have found that when cancer cells and the like are cultured in suspension in a medium composition capable of culturing cells and tissues while keeping the suspended state without substantially increasing the viscosity of the liquid medium, the cell proliferation is more promoted in comparison to the case with adhesion culture on a plate (patent document 1). They have found that, by culturing vascular smooth muscle cells in suspension in the medium composition capable of culturing cells and tissues while keeping the suspended state, cell cycle is unexpectedly arrested while maintaining good viability, cell proliferation is suppressed, and the cells can be maintained for a long term while avoiding the risk of property change.

Based on such finding, they have conducted further studies and completed the present invention.

Therefore, the present invention provides the following:

[1] A method for culturing vascular smooth muscle cells, which comprises culturing vascular smooth muscle cells in suspension in a medium composition comprising deacylated gellan gum.
[2] The method according to [1], wherein the vascular smooth muscle cells in a cell proliferation-arrested state are cultured in suspension.
[3] A method for suppressing proliferation of vascular smooth muscle cells, which comprises culturing vascular smooth muscle cells in suspension in a medium composition comprising deacylated gellan gum.
[4] A method for preserving vascular smooth muscle cells, which comprises suspending vascular smooth muscle cells in a medium composition comprising deacylated gellan gum.
[5] The method according to any one of [1] to [4] wherein the medium composition comprises deacylated gellan gum at a concentration of 0.005 - 0.3% (w/v).

[Effect of the Invention]

According to the present invention, vascular smooth muscle cells can be maintained in vitro for a long term while suppressing cell proliferation. According to the present invention, vascular smooth muscle cells can be transported under conditions free of a freezing operation, and the property change of cells observed when freezing or adhering them is expected to be minimized. The present invention contributes to the development of regenerative medicine using vascular smooth muscle cells.

[Brief Description of the Drawings]

[Fig. 1] Analysis of cell-cycle related protein expression in vascular smooth muscle cells by Western blotting.
[Fig. 2] Analysis of cell-cycle related protein expression in vascular smooth muscle cells by Western blotting.
[Fig. 3] Analysis of cell-cycle related protein expression in NIH3T3 cells by Western blotting.
[Fig. 4] Analysis of cell-cycle related protein expression in vascular smooth muscle cells by Western blotting.
[Fig. 5] Analysis of cell-cycle related protein expression in NIH3T3 cells by Western blotting.
[Fig. 6] Time-course changes of proliferation and survival rate of vascular smooth muscle cells. A: total cell number, B: survival rate. Open circle: cells subjected to adhesion culture on a plate were seeded on a plate and further subjected to adhesion culture. Filled circle: cells cultured in suspension in deacylated gellan gum-containing medium were seeded on a plate and subjected to adhesion culture.

[Description of Embodiments]

(1) Medium composition to be used in the present invention

The medium composition to be used in the present invention is a medium composition comprising deacylated gellan gum. The medium composition enables culturing desired cells (i.e., vascular smooth muscle cells) or a tissue

containing the same while maintaining the suspended state. The medium composition can be prepared according to the descriptions of WO 2014/017513 A1 and US 2014/0106348 A1. In the following, the medium composition is sometimes referred to as medium composition I.

A cell is a most basic unit constituting animals, which has, as its elements, cytoplasm and various organelles inside the cellular membrane.

Suspending of cells and/or tissues in the present invention refers to a state where cells and/or tissues do not adhere to a culture vessel (non-adhesive). Furthermore, in the present invention, when the cells and/or tissues are proliferated, differentiated or maintained, the state where the cells and/or tissues are uniformly dispersed and suspended in the liquid medium composition in the absence of a pressure on or vibration of the liquid medium composition from the outside or shaking, rotating operation and the like in the composition is referred to as "static suspension", and cultivation of the cells and/or tissues in such condition is referred to as "static suspension culture". In the "static suspension", the period of suspending includes not less than 5 min, not less than 1 hr, not less than 24 hr, not less than 48 hr, not less than 7 days etc., though the period is not limited thereto as long as the suspended state is maintained.

The medium composition to be used in the present invention permits static suspension of cells and/or tissues at least on one point in the temperature range (e.g., 0 - 40°C) capable of maintaining or culturing cells or tissues. The medium composition to be used in the present invention permits static suspension of cells and/or tissues at least at one point in the temperature range of preferably 25 - 37°C, most preferably 37°C.

Whether or not static suspension is possible can be evaluated by, for example, uniformly dispersing the cells to be cultured in a medium composition to be evaluated at a concentration of $2\times10^4$ cells/ml, injecting 10 ml thereof in a 15 ml conical tube, standing the tube for at least not less than 5 min (e.g., not less than 1 hr, not less than 24 hr, not less than 48 hr, not less than 7 days) at 37°C, and observing whether the suspended state of the cells is maintained. When not less than 70% of the total cells are in a suspended state, it is concluded that the suspended state was maintained. Polystyrene beads (Size 500-600 μm, manufactured by Polysciences Inc.) may be used for evaluation instead of the cells.

The medium composition to be used in the present invention is a composition comprising deacylated gellan gum.

The medium composition to be used in the present invention is preferably a composition permitting recovery of the cells or tissues from the medium composition when exchanging the medium composition during culture, and after completion of the culture. More preferably, it is a composition that does not require any of a temperature change, a chemical treatment, an enzymatic treatment and a shear force when recovering the cells or tissues from the medium composition.

The structure in the medium composition of the present disclosure is formed from a particular compound and exerts an effect of suspending cells and/or tissues uniformly. More particularly, it includes polymer compounds assembled via an ion, a three-dimensional network formed by polymer compounds and the like. It is known that polysaccharides form a microgel via a metal ion (e.g., JP-A-2004-129596), and the structure of the present disclosure also includes such microgel as one aspect. One aspect of the polymer compounds assembled of via an ion is a film structure.

The size of the structure in the present invention is preferably a size that passes a filter having a pore size of 0.2 μm to 200 μm when it is passed through a filter. The lower limit of the pore size is more preferably more than 1 μm and, in consideration of stable suspension of cells or tissues, it more preferably exceeds 5 μm. The upper limit of the pore size is more preferably 100 μm or less and, in consideration of the size of the cells or tissues, it is more preferably 70 μm or less.

The particular compound in the present invention (deacylated gellan gum) refers to a compound that forms, upon mixing with a liquid medium, an indeterminate structure which is uniformly dispersed in the liquid, substantially retains the cells and/or tissues without substantially increasing the viscosity of the liquid, and exerts an effect of preventing sediment thereof. The "without substantially increasing the viscosity of the liquid" means that the viscosity of the liquid does not exceed 8 mPa•s. In this case, the viscosity of the liquid (that is, the viscosity of the medium composition to be used in the present invention) is not more than 8 mPa.s, preferably not more than 4 mPa•s, more preferably not more than 2 mPa•s, at 37°C. Furthermore, the chemical structure, molecular weight, property etc. of the particular compound are not limited as long as it forms the structure in a liquid medium, and exerts an effect of uniformly suspending (preferably statically suspending) the cells and/or tissues without substantially increasing the viscosity of the liquid.

The viscosity of the liquid containing the structure can be measured, for example, by the method described in the below-mentioned Examples. Specifically, it can be measured under 37°C conditions and using an E-type viscosity meter (manufactured by Toki Sangyo Co., Ltd., TV-22 type viscosity meter, model: TVE-22L, corn roter: standard roter 1°34'×R24, rotating speed 100 rpm).

Examples of the particular compound to be used in the present disclosure include, but are not limited to, polymer compounds, preferably a polymer compound having an anionic functional group.

As the anionic functional group, a carboxy group, sulfo group, phosphate group and a salt thereof can be mentioned, with preference given to carboxy group or a salt thereof.

As a polymer compound to be used in the present disclosure, one having one or more kinds selected from the

aforementioned anionic functional groups can be used.

Specific preferable examples of the polymer compound to be used in the present disclosure include, but are not limited to, polysaccharides wherein not less than 10 monosaccharides (e.g., triose, tetrose, pentose, hexose, heptose etc.) are polymerized, more preferably, acidic polysaccharides having an anionic functional group. The acidic polysaccharides here is not particularly limited as long as it has an anionic functional group in the structure thereof, and includes, for example, polysaccharides having a uronic acid (e.g., glucuronic acid, iduronic acid, galacturonic acid, mannuronic acid), polysaccharides having a sulfate group or phosphate group in a part of the structure thereof, and polysaccharides having the both structures, and includes not only naturally-obtained polysaccharides but also polysaccharides produced by microorganisms, polysaccharides produced by genetic engineering, and polysaccharides artificially synthesized using an enzyme. More specifically, examples thereof include polymer compounds composed of one or more kinds selected from the group consisting of hyaluronic acid, gellan gum, deacylated gellan gum (hereinafter sometimes to be referred to as DAG), rhamsan gum, diutan gum, xanthan gum, carageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate and a salt thereof. Polysaccharides are preferably hyaluronic acid, DAG, diutan gum, xanthan gum, carageenan or a salt thereof, most preferably DAG since in consideration of the ability to suspend cells or tissues at a low concentration and ease in recovering the cells or tissues.

The salt here includes, for example, salts with alkali metal such as lithium, sodium, potassium, salts with alkaline earth metals such as calcium, barium, magnesium, and salts with aluminum, zinc, copper, iron, ammonium, organic base and amino acid and the like.

The weight average molecular weight of these polymer compounds (polysaccharides etc.) is preferably 10,000 to 50,000,000, more preferably 100,000 to 20,000,000, still more preferably 1,000,000 to 10,000,000. For example, the molecular weight can be measured based on pullulan by gel penetration chromatography (GPC).

Furthermore, phosphorylated DAG can also be used. The phosphorylation can be performed by a known method.

In the present invention, plural kinds (preferably two kinds) of the above-mentioned polysaccharides can be used in combination. The kind of the combination of the polysaccharides is not particularly limited as long as the aforementioned structure is formed in a liquid medium, the cells and/or tissues can be uniformly suspended (preferably suspended statically) without substantially increasing the viscosity of the liquid, and the combination includes at least DAG or a salt thereof. That is, the combination of polysaccharides contains DAG or a salt thereof, and polysaccharides other than DAG and a salt thereof (e.g., xanthan gum, alginic acid, carageenan, diutan gum, methylcellulose, locust bean gum or a salt thereof). Examples of specific combination of polysaccharides include, but are not limited to, DAG and rhamsan gum, DAG and diutan gum, DAG and xanthan gum, DAG and carageenan, DAG and xanthan gum, DAG and locust bean gum, DAG and κ-carageenan, DAG and sodium alginate, DAG and methylcellulose and the like.

More specific preferable examples of the particular compound to be used in the present disclosure include hyaluronic acid, deacylated gellan gum, diutan gum, carageenan and xanthan gum and a salt thereof. Most preferable examples include deacylated gellan gum and a salt thereof, since the viscosity of the medium composition can be made low and the cells or tissues can be easily recovered.

The deacylated gellan gum used in the present invention is a linear polymer polysaccharide containing 4 molecules of sugars of 1-3 bonded glucose, 1-4 bonded glucuronic acid, 1-4 bonded glucose and 1-4 bonded rhamnose as the constituent unit, which is a polysaccharide of the following formula (I) wherein $R_1$, $R_2$ are each a hydrogen atom, and n is an integer of two or more. $R_1$ may contain a glyceryl group, $R_2$ may contain an acetyl group, and the content of the acetyl group and glyceryl group is preferably not more than 10%, more preferably not more than 1%.

The DAG in the present invention takes various forms. It uptakes a metal ion (e.g., calcium ion) in a liquid medium when mixed with the liquid medium, forms an indeterminate structure via the metal ion, and suspends the cells and/or tissues. The viscosity of the medium composition to be used in the present invention prepared from deacylated gellan gum is not more than 8 mPa•s, preferably not more than 4 mPa•s, and more preferably not more than 2 mPa•s in view of ease in recovering the cells or tissues.

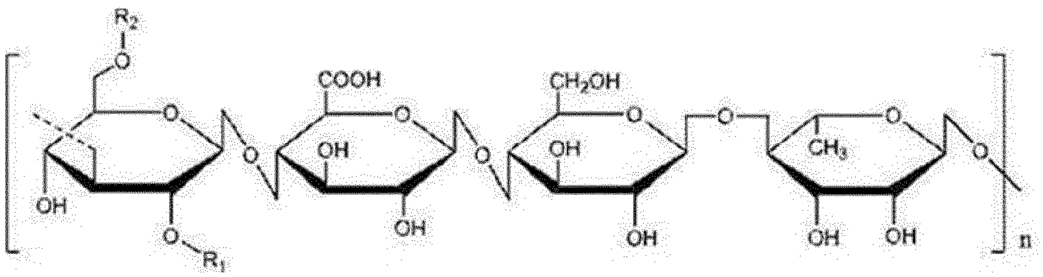

(I)

The particular compound in the present invention (DAG) can be obtained by a chemical synthesis method. When the compound is a naturally-occurring substance, it is preferably obtained from various plants, various animals, various microorganisms containing the compound by extraction, separation and purification by conventional techniques. For extraction, the compound can be extracted efficiently by using water or supercritical gas. For example, as a production method of gellan gum, a producing microorganism is cultured in a fermentation medium, a mucosal substance produced outside the cell is recovered by a general purification method and, after the steps of drying, pulverizing and the like, it is powderized. For deacylated gellan gum, an alkali treatment is applied when a mucousal substance is recovered, the glyceryl group and the acetyl group bonded to 1-3 bonded glucose residue are deacylated and recovered. Examples of the purification method include liquid-liquid extraction, fractional precipitation, crystallization, various kinds of ion exchange chromatography, gel filtration chromatography using Sephadex LH-20 and the like, adsorption chromatography using activated carbon, silica gel and the like, adsorption and desorption treatment of active substance by thin layer chromatography, high performance liquid chromatography using reversed-phase column and the like, and impurity can be removed and the compound can be purified by using them singly or in combination in any order, or repeatedly. Examples of the gellan gum-producing microorganism include, but are not limited to, Sphingomonas elodea and microorganism obtained by genetically modification of Sphingomonas elodea.

For deacylated gellan gum, commercially available products, for example, "KELCOGEL (registered trade mark of CP Kelco) CG-LA" manufactured by SANSHO Co., Ltd., "KELCOGEL (registered trade mark of CP Kelco)" manufactured by San-Ei Gen F.F.I., Inc. and the like can be used. As native-type gellan gum, "KELCOGEL (registered trade mark of CP Kelco) HT" manufactured by San-Ei Gen F.F.I., Inc. and the like can be used.

The concentration of the particular compound in a medium depends on the kind of the particular compound, and can be appropriately determined within the range where the particular compound can form the aforementioned structure in a liquid medium, and can uniformly suspend (preferably statically suspend) the cells and/or tissues without substantially increasing the viscosity of the liquid. It is generally 0.0005% to 1.0% (w/v), preferably 0.001% to 0.4% (w/v), more preferably 0.005% to 0.1% (w/v), still more preferably 0.005% to 0.05% (w/v). For example, in the case of deacylated gellan gum, as in the invention, it is added to a medium at 0.001% to 1.0% (w/v), preferably 0.003% to 0.5% (w/v), more preferably 0.005% to 0.3% (w/v), more preferably 0.01% to 0.05% (w/v), most preferably, 0.01% to 0.03% (w/v). In the case of xanthan gum, it is added to a medium at 0.001% to 5.0% (w/v), preferably 0.01% to 1.0% (w/v), more preferably 0.05% to 0.5% (w/v), most preferably 0.1% to 0.2% (w/v). In the case of a κ-carageenan and locust bean gum mixture, it is added to a medium at 0.001% to 5.0% (w/v), preferably 0.005% to 1.0% (w/v), more preferably 0.01% to 0.1% (w/v), most preferably 0.03% to 0.05% (w/v). In the case of native gellan gum, it is added to a medium at 0.05% to 1.0% (w/v), preferably 0.05% to 0.1% (w/v).

When plural kinds (preferably two kinds) of the above-mentioned polysaccharides are used in combination, the concentration of the polysaccharides can be appropriately set as long as they form the aforementioned structure in a liquid medium, and can uniformly suspend (preferably statically suspend) the cells and/or tissues without substantially increasing the viscosity of the liquid. For example, when a combination of DAG or a salt thereof and polysaccharide other than DAG and a salt thereof is used, the concentration of DAG or a salt thereof is, for example, 0.005 - 0.02% (w/v), preferably 0.01 - 0.02% (w/v), and the concentration of a polysaccharide other than DAG and a salt thereof is, for example, 0.0001 - 0.4% (w/v), preferably 0.005 - 0.4% (w/v), more preferably 0.1 - 0.4% (w/v). Specific examples of the combination of the concentration range include the following. DAG or a salt thereof: 0.005 - 0.02% (preferably 0.01 - 0.02%) (w/v)
polysaccharide other than DAG
xanthan gum: 0.1 - 0.4% (w/v)
sodium alginate: 0.0001 - 0.4% (w/v) (preferably 0.1 - 0.4% (w/v))
native gellan gum: 0.0001 - 0.4% (w/v)
locust bean gum: 0.1 - 0.4% (w/v)
methylcellulose: 0.1 - 0.4% (w/v) (preferably 0.2 - 0.4% (w/v)) carageenan: 0.05 - 0.1% (w/v)
diutan gum: 0.05 - 0.1% (w/v)

The concentration can be calculated by the following formula.

$$\text{Concentration } [\% \text{ (w/v)}] = \text{weight (g) of particular compound/ volume (ml) of medium composition} \times 100$$

The aforementioned compound can also be further converted to a different derivative by a chemical synthesis method, and the thus-obtained derivative can also be used effectively in the present invention. Specifically, in the case of deacylated gellan gum, as in the invention, a derivative of a compound represented by the formula (I) wherein a hydroxyl group for $R_1$ and/or $R_2$ is substituted by $C_{1-3}$ alkoxy group, $C_{1-3}$ alkylsulfonyl group, a monosaccharide residue

such as glucose, fructose and the like, oligosaccharide residue such as sucrose, lactose and the like, or amino acid residue such as glycine, arginine and the like can also be used in the present invention. In addition, the compound can also be crosslinked using a crosslinking agent such as 1-ethyl-3-(3-dimethylaminopropyl)carbodiimide (EDC) and the like.

The DAG or salt thereof to be used in the present invention can be present in any crystal form depending on the production conditions, and can be present as any hydrate. Such crystal form, hydrate and mixtures thereof are also encompassed in the scope of the present invention. In addition, they may be present as a solvate containing an organic solvent such as acetone, ethanol, tetrahydrofuran and the like. Such forms are all encompassed in the scope of the present invention.

The DAG to be used in the present invention may be present in the form of tautomer formed by isomerization in the ring or outside the ring, geometric isomer or tautomer, or a mixture of geometric isomers, or mixtures thereof. When the DAG of the present invention has an asymmetric center, irrespective of whether the compound is formed by isomerization, it may be present in the form of a resolved optical isomer or a mixture containing same at any ratio.

The medium composition to be used in the present invention may contain a metal ion, for example, a divalent metal ion (calcium ion, magnesium ion, zinc ion, ferrous ion, copper ion etc.), and preferably contains calcium ion. Two or more kinds of metal ions can be used in combination, for example, calcium ion and magnesium ion, calcium ion and zinc ion, calcium ion and ferrous ion, and calcium ion and copper ion. Those of ordinary skill in the art can appropriately determine the combination. In one embodiment, since the medium composition contains a metal ion (e.g., calcium ion), polymer compounds are assembled via the metal ion to form a three-dimensional network (e.g., polysaccharides form a microgel via a metal ion (e.g., calcium ion)), whereby the structure of the present invention can be formed. The concentration of the metal ion can be appropriately set within the range where the particular compound can form the aforementioned structure in a liquid medium, and can uniformly suspend (preferably statically suspend) the cells and/or tissues without substantially increasing the viscosity of the liquid medium. The metal ion concentration is, but is not limited to, 0.1 mM - 300 mM, preferably 0.5 mM - 100 mM. The metal ion may be mixed with a medium, or a salt solution is separately prepared and added to the medium. The medium composition to be used in the present invention may contain the below-mentioned extracellular matrix, adhesion molecule and the like.

When cells and/or tissues are cultured in vitro, the DAG to be used in the present invention exerts an effect of suspending (preferably effect of statically suspending) the cells and/or tissues in a liquid containing the structure composed of the particular compound. By the suspending effect, a more increased number of the cells and/or tissues per a given volume can be cultivated as compared to a monolayer culture. When a conventional suspension culture method accompanies rotation or shaking operation, the proliferation rate and recovery rate of the cells and/or tissues may become low, or the function of the cells may be impaired since a shear force acts on the cells and/or tissues. The medium composition of the present invention, which contains DAG, can uniformly disperse the cells and/or tissues without an operation such as shaking and the like, and can obtain the object cells and/or tissues easily in a large amount without loss of the cell function. In addition, when cells and/or tissues are cultured in suspension in a conventional medium containing a gel substrate, observation or recovery of the cells and/or tissues is sometimes difficult, and the function thereof is sometimes impaired during recovery. However, using the medium composition containing DAG, the cells and/or tissues can be subjected to suspension culture, observed without impairment of the function thereof, and can be recovered. In addition, a conventional medium containing a gel substrate sometimes shows high viscosity that makes it difficult to exchange the medium. However, since the medium composition containing the structure composed of the particular compound of the present invention has low viscosity, it can be exchanged easily with a pipette, pump and the like.

When cells and/or tissues are cultivated using the DAG in the present invention, a medium composition can be prepared by mixing a medium used for cultivating the cells and/or tissues and the DAG. According to the classification by such composition of the medium, natural medium, semisynthetic medium and synthetic medium can be mentioned. According to the classification by the form, semi-solid medium, liquid medium, powder medium (hereinafter sometimes to be referred to as powder medium) and the like can be mentioned. When the cells and/or tissues are derived from an animal, any medium used for culturing animal cells can be used. Examples of the medium include Dulbecco's Modified Eagle's Medium (DMEM), HamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), StemPro hESC SFM (manufactured by Invitrogen), Essential8 (registered trade mark) medium (manufactured by Gibco), mTeSR1 or 2 medium (manufactured by STEMCELL Technologies), ReproFF or ReproFF2 (manufactured by ReproCELL), PSGro hESC/iPSC medium (manufactured by System Biosciences), NutriStem (registered trade mark) medium (manufactured by Biological Industries), CSTI-7 medium (manufactured by Cell Science & Technology Institute, Inc.), MesenPRO RS medium (manufactured by Gibco), MF-Medium (registered

trade mark) mesenchymal stem cell proliferation medium (manufactured by TOYOBO CO., LTD.), Sf-900II (manufactured by Invitrogen), Opti-Pro (manufactured by Invitrogen), and the like.

The medium to be used for culture of vascular smooth muscle cells can be the above-mentioned medium added with a cell adhesion factor, and examples thereof include Matrigel, collagen gel, gelatin, poly-L-lysine, poly-D-lysine, laminin and fibronectin. It is possible to add two or more kinds of these cell adhesion factors in combination. Furthermore, a medium to be used for culture of vascular smooth muscle cells can be further mixed with a thickener such as guargum, tamarind gum, alginic acid propylene glycol, locust bean gum, gum arabic, tara gum, tamarind gum, methylcellulose and the like.

Those of ordinary skill in the art can freely add, according to the object, sodium, potassium, calcium, magnesium, phosphorus, chlorine, various amino acids, various vitamins, antibiotic, serum, fatty acid, sugar and the like to the above-mentioned medium. For culture of animal-derived cells and/or tissues, those of ordinary skill in the art can also add, according to the object, one or more kinds of other chemical components and biogenic substances in combination.

Examples of the components to be added to a medium for animal-derived cells and/or tissues include fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, various vitamins, various amino acids, agar, agarose, collagen, methylcellulose, various cytokines, various hormones, various growth factors, various extracellular matrices, various cell adhesion molecules and the like.

Examples of the antibiotic to be added to a medium include sulfa drug, penicillin, phenethicillin, methicillin, oxacillin, cloxacillin, dicloxacillin, flucloxacillin, nafcillin, ampicillin, penicillin, amoxicillin, ciclacillin, carbenicillin, ticarcillin, piperacillin, azlocillin, mezlocillin, mecillinam, andinocillin, cephalosporin and a derivative thereof, oxolinic acid, amifloxacin, temafloxacin, nalidixic acid, Piromidic acid, ciprofloxacin, cinoxacin, norfloxacin, perfloxacin, Rosaxacin, ofloxacin, enoxacin, pipemidic acid, sulbactam, clavulanic acid, β-bromopenisillanic acid, β-chloropenisillanic acid, 6-acetylmethylene-penisillanic acid, cephoxazole, sultampicillin, adinoshirin and sulbactam formaldehyde hudrate ester, tazobactam, aztreonam, sulfazethin, isosulfazethin, norcardicin, m-carboxyphenol, phenylacetamidophosphonic acid methyl, Chlortetracycline, oxytetracycline, tetracycline, demeclocycline, doxycycline, methacycline, and minocycline.

When the particular compound in the present invention (DAG) is added to the above-mentioned medium, the particular compound is dissolved or dispersed in an appropriate solvent when in use (this is used as a medium additive). Thereafter, the medium additives can be added to a medium such that the concentration of the particular compound in the medium is, as described above in detail, a concentration at which the cells and/or tissues can be uniformly suspended (preferably statically suspended) without substantially increasing the viscosity of the liquid medium, for example, 0.0005% to 1.0% (w/v), preferably 0.001% to 0.4% (w/v), more preferably 0.005% to 0.1% (w/v), further preferably 0.005% to 0.05% (w/v). For example, in the case of deacylated gellan gum, it is added to a medium at 0.001% to 1.0% (w/v), preferably 0.003% to 0.5% (w/v), more preferably 0.005% to 0.3% (w/v), most preferably 0.01% to 0.05% (w/v). In the case of xanthan gum, it is added to a medium at 0.001% to 5.0% (w/v), preferably 0.01% to 1.0% (w/v), more preferably 0.05% to 0.5% (w/v), most preferably 0.1% to 0.2% (w/v). In the case of a κ-carageenan and locust bean gum mixture, it is added to a medium at 0.001% to 5.0% (w/v), preferably 0.005% to 1.0% (w/v), more preferably 0.01% to 0.1%, most preferably 0.03% to 0.05% (w/v), in a total of the both compounds. In the case of a deacylated gellan gum and diutan gum mixture, it is added to a medium at 0.001% to 1.0% (w/v), most preferably 0.005% to 0.01% (w/v), in a total of the both compounds. In the case of a deacylated gellan gum and methylcellulose mixture, it is added to a medium at 0.001% to 1.0% (w/v), most preferably 0.005% to 0.2% (w/v), in a total of the both compounds. In the case of a deacylated gellan gum and locust bean gum mixture, it is added to a medium at 0.001% to 1.0% (w/v), most preferably 0.01% to 0.1% (w/v), in a total of the both compounds. In the case of a deacylated gellan gum and sodium alginate mixture, it is added to a medium at 0.001% to 1.0% (w/v), most preferably 0.01% to 0.1% (w/v), in a total of the both compounds. In the case of a deacylated gellan gum and xanthan gum mixture, it is added to a medium at 0.001% to 1.0% (w/v), most preferably 0.01% to 0.1% (w/v), in a total of the both compounds. In the case of a deacylated gellan gum and κ-carageenan mixture, it is added to a medium at 0.001% to 1.0% (w/v), most preferably 0.01% to 0.1% (w/v), in a total of the both compounds. The concentration can be calculated by the following formula.

$$\text{Concentration [\% (w/v)]} = \text{weight (g) of particular compound/volume (ml) of medium composition} \times 100$$

Here, examples of appropriate solvent used for the medium additive include, but are not limited to, aqueous solvents such as water, dimethyl sulfoxide (DMSO), various alcohols (e.g., methanol, ethanol, butanol, propanol, glycerol, propylene glycol, butyleneglycol and the like), and the like. In this case, the concentration of the particular compound is 0.001% to 5.0% (w/v), preferably 0.01% to 1.0% (w/v), more preferably 0.1% to 0.6% (w/v). It is also possible to further add an additive to enhance the effect of the particular compound, or lower the concentration when in use. As an example

of such additive, one or more kinds of polysaccharides such as guargum, alginic acid propylene glycol ester, locust bean gum, gum arabic, tara gum, tamarind gum, methylcellulose, carboxymethylcellulose, agarose, tamarind seed gum, pullulan and the like can be added. It is also possible to immobilize the particular compound on the surface of a carrier or carry the particular compound inside a carrier during culture. The particular compound can have any form during provision or preservation. The particular compound may be in the form of a formulated solid such as tablet, pill, capsule, granule, or a liquid such as a solution obtained by dissolving in an appropriate solvent using a solubilizer or a suspension, or may be bonded to a basal plate or a single substance. Examples of the additive used for formulation include preservatives such as p-hydroxybenzoic acid esters and the like; excipients such as lactose, glucose, sucrose, mannit and the like; lubricants such as magnesium stearate, talc and the like; binders such as polyvinyl alcohol, hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art. The particular compound of the present invention may be sterilized as necessary. The sterilization method is not particularly limited, and, for example, radiation sterilization, ethylene oxide gas sterilization, autoclave sterilization, filter sterilization and the like can be mentioned. When filter sterilization (hereinafter sometimes to be referred to as filtration sterilization) is to be performed, the material of the filter part is not particularly limited and, for example, glass fiber, nylon, PES (polyethersulfone), hydrophilic PVDF (polyvinylidene fluoride), cellulose mixed ester, celluloseacetate, polytetrafluoroethylene and the like can be mentioned. While the size of the pore in the filter is not particularly limited, it is preferably 0.1 μm to 10 μm, more preferably 0.1 μm to 1 μm, most preferably 0.1 μm to 0.5 μm. The sterilization treatment may be applied when the particular compound is in a solid state or a solution state.

The medium composition of the present invention can be obtained by forming the above-mentioned structure in a liquid medium by adding a solution or dispersion of the particular compound prepared above to the liquid medium. Since a medium generally contains a sufficient concentration of metal ions (e.g., divalent metal ions such as calcium ion and the like) to assemble polymer compounds or form a three-dimensional network of polymer compounds via ions, the medium composition to be used in the present invention can be obtained by simply adding a solution or dispersion of the particular compound of the present invention to a liquid medium. Alternatively, a medium may be added to the medium additive (the solution or dispersion of the particular compound). Furthermore, the medium composition to be used in the present invention can also be prepared by mixing the particular compound and medium components in an aqueous solvent (e.g., water including ion exchange water, ultrapure water and the like). Examples of the embodiment of mixing include, but are not limited to, (1) mixing a liquid medium and a medium additive (solution), (2) mixing the above-mentioned polymer compound (solid such as powder etc.) to a liquid medium, (3) mixing a powder medium to a medium additive (solution), (4) mixing a powder medium and the above-mentioned polymer compound (solid such as powder etc.) to an aqueous solvent, and the like. To prevent the particular compound in the medium composition to be used in the present invention from being non-uniformly distributed, the embodiment of (1) or (4), or (1) or (3) is preferable.

When the particular compound is dissolved in a solvent (e.g., aqueous solvent such as water, liquid medium) or the particular compound and a powder medium are dissolved in a solvent, the mixture is preferably heated to promote dissolution. The heating temperature is, for example, 80°C - 130°C, preferably 100°C - 125°C (e.g., 121°C) at which heating sterilization is performed. After heating, the obtained solution of the particular compound is cooled to room temperature. The above-mentioned structure composed of the particular compound can be formed by adding the aforementioned metal ion (e.g., divalent metal ions such as calcium ion and the like) to the solution (e.g., by adding the solution to a liquid medium). Alternatively, the above-mentioned structure composed of the particular compound can also be formed by heating (e.g., 80°C - 130°C, preferably 100°C - 125°C (e.g., 121°C)) the particular compound when dissolved in a solvent (e.g., an aqueous solvent such as water and liquid medium) containing the aforementioned metal ion (e.g., divalent metal ions such as calcium ion and the like), and cooling the obtained solution to room temperature.

Examples of the preparation method of the medium composition to be used in the present invention are shown below, which are not to be construed as limitative. The particular compound is added to ion exchange water or ultrapure water. Then, the mixture is stirred at a temperature at which the particular compound can be dissolved (e.g., not less than 60°C, not less than 80°C, not less than 90°C) to allow for dissolution to a transparent state. After dissolving, the mixture is allowed to cool with stirring, and sterilized (e.g., autoclave sterilization at 121°C for 20 min). After cooling to room temperature, the aforementioned sterilized aqueous solution is added with stirring (e.g., homomixer etc.) to a given medium to be used for static culture to uniformly mix the solution with the medium. The mixing method of the aqueous solution and the medium is not particularly limited, and may be manual mixing such as pipetting etc., or mixing with an instrument such as magnetic stirrer, mechanical stirrer, homomixer and homogenizer. Furthermore, the medium composition to be used in the present invention can be filtrated through a filter after mixing. The size of the pore of the filter to be used for the filtration treatment is 5 μm to 100 μm, preferably 5 μm to 70 μm, more preferably 10 μm to 70 μm.

Alternatively, a powder medium and the above-mentioned polymer compound (solid such as powder and the like) are mixed with an aqueous solvent, and the mixture is heated at the above-mentioned temperature to give a medium composition to be used in the present invention.

For example, when deacylated gellan gum is prepared, deacylated gellan gum is added to ion exchange water

or ultrapure water at 0.1% to 1% (W/V), preferably 0.2% to 0.5% (W/V), more preferably 0.3% to 0.4% (W/V). In an aspect, when deacylated gellan gum is prepared, deacylated gellan gum is added to ion exchange water or ultrapure water at 0.1% to 1% (W/V), preferably 0.2% to 0.8% (W/V), more preferably 0.3% to 0.6% (W/V).

Then, the aforementioned deacylated gellan gum is dissolved to a transparent state by stirring at any temperature as long as dissolution is possible, which may be, for example, not less than 60°C, preferably not less than 80°C, more preferably not less than 90°C (e.g., 80 to 130°C) . After dissolution, the mixture is allowed to cool with stirring, and sterilized with autoclave at, for example, 121°C for 20 min. After cooling to room temperature, for example, the aqueous solution is added to a liquid medium such as DMEM/F-12 medium with stirring by a homomixer and the like to a desired final concentration (e.g., when the final concentration is 0.015%, the ratio of 0.3% aqueous solution:medium is 1:19), and the mixture is homogeneously mixed. The mixing method of the aqueous solution and the medium is not particularly limited, and may be manual mixing such as pipetting etc., or mixing with an instrument such as magnetic stirrer, mechanical stirrer, homomixer and homogenizer. Furthermore, the medium composition to be used in the present invention can be filtrated through a filter after mixing. The size of the pore of the filter to be used for the filtration treatment is 5 $\mu$m to 100 $\mu$m, preferably 5 $\mu$m to 70 $\mu$m, more preferably 10 $\mu$m to 70 $\mu$m.

Preferable embodiments of the medium composition to be used in the present invention and a production method thereof are described below.

The medium composition to be used in the present invention is preferably a medium composition which enables culture of cells or tissues in suspension, which is characterized in that the medium composition has a viscosity of the aforementioned medium composition of not more than 8 mPa•s (under 37°C conditions), and contains deacylated gellan gum or a salt thereof. In one embodiment, the concentration of deacylated gellan gum or a salt thereof in the medium composition is 0.005 - 0.3%(w/v) (preferably, 0.01 - 0.05%(w/v)). In one embodiment, the medium composition further contains a polysaccharide other than deacylated gellan gum or a salt thereof. In one embodiment, the medium composition contains divalent metal ions (e.g., calcium ion) at a concentration sufficient for deacylated gellan gum to form the structure which enables culture of cells or tissues in suspension. The concentration is, for example, 0.1 mM to 300 mM, preferably, 0.5 mM to 100 mM.

The medium composition can be produced by mixing deacylated gellan gum or a salt thereof and a medium. In one embodiment, the medium is a liquid medium. In one embodiment, the liquid medium contains divalent metal ions (e.g., calcium ion) at a concentration sufficient for deacylated gellan gum to form the structure which enables culture of cells or tissues in suspension. The concentration is, for example, 0.1 mM to 300 mM, preferably, 0.5 mM to 100 mM. In one embodiment, deacylated gellan gum or a salt thereof dissolved or dispersed in a solvent and a medium are mixed. In one embodiment, deacylated gellan gum or a salt thereof dissolved or dispersed in a solvent is sterilized. In one embodiment, sterilization is performed by autoclave sterilization. In another embodiment, sterilization is performed by filtration sterilization. In one embodiment, filtration sterilization is performed by passing through a 0.1 - 0.5 $\mu$m filter.

(2) Culture of vascular smooth muscle cell

The present invention provides an in vitro method for culturing vascular smooth muscle cells in suspension in the medium composition I. By subjecting the vascular smooth muscle cells to a suspension culture in the medium composition I, the cell cycle is arrested while maintaining good viability, cell proliferation is suppressed, and the cells can be maintained for a long term while avoiding the risk of property change. The present invention also encompasses such method of suppressing cell proliferation of vascular smooth muscle cells, a method of suppressing property change of vascular smooth muscle cells, and a method of maintaining viability of vascular smooth muscle cells in cell proliferation arrest (method of suppressing decrease in survival rate).

The vascular smooth muscle cells to be used in the present invention are vascular smooth muscle cells of mammals. Examples of the mammal include rodents such as mouse, rat, hamster, guinea pig and the like, lagomorphs such as rabbit and the like, ungulates such as swine, bovine, goat, horse, sheep and the like, carnivora such as dog, cat and the like, primates such as human, monkey, Macaca mulatta, Macaca fascicularis, marmoset, orangutan, chimpanzee and the like, and the like. The mammal is preferably rodent (mouse etc.), lagomorph or primate (human etc.), preferably human.

The vascular smooth muscle cells to be used in the present invention are preferably isolated. "Isolation" means that an operation to remove factors other than the object components or cells has been performed, and the state of natural presence has passed. The purity of the "isolated vascular smooth muscle cells" (percentage of the cell number of vascular smooth muscle cells in the total cell number) is generally not less than 70%, preferably not less than 80%, more preferably not less than 90%, further preferably not less than 99%, most preferably 100%. In one embodiment, the percentage of the cell number of vascular smooth muscle cells in the total cells contained in the culture in the culture method of the present invention is generally not less than 70%, preferably not less than 80%, more preferably not less than 90%, further preferably not less than 99%, most preferably 100%.

For example, vascular smooth muscle cells can be isolated from the living body by detaching tunica media

alone from the artery, adhering same to the bottom surface of a plate, and culturing same for 1 - 2 weeks, or by treating the tunica media of the blood vessel with collagenase or elastase. An isolation method of vascular smooth muscle cells is described in scientific articles (May-Jun et al, 1984; Arteriosclerosis, 4(3):183-188, Orekhov et al, Med Biol. 1984; 62(4):255-259.), and well known in the technical field. A commercially available vascular smooth muscle cells may also be used.

In one embodiment, the vascular smooth muscle cells to be used in the present invention are primary cultured cells. The primary cultured cell means a cell separated from the living body and prior to a passage operation. When the culture method of the present invention is used, since the cell cycle of vascular smooth muscle cells is arrested while maintaining good viability, cell proliferation is suppressed, and the cells can be maintained for a long term while avoiding the risk of property change, vascular smooth muscle cells in the primary culture can be cultured for a long term while maintaining the properties in the body.

For suspension culture of vascular smooth muscle cells, culture vessels generally used for cell culture such as schale, flask, plastic bag, Teflon (registered trade mark) bag, dish, schale, dish for tissue culture, multidish, microplate, microwell plate, multiplate, multiwell plate, chamber slide, cell culture flask, spinner flask, tube, tray, culture bag, roller bottle and the like can be used for cultivation. To reduce the risk that a part of the vascular smooth muscle cells to be cultured adheres to a culture vessel, proliferates and causes property change, in one embodiment, the culture vessel to be used for the culture method of the present invention is non-cell-adhesive. As a non-cell-adhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used.

Those of ordinary skill in the art can freely select the form and state of the vascular smooth muscle cells to be cultured. Specific preferable examples thereof include, but are not particularly limited to, a state in which vascular smooth muscle cells are dispersed as single cells in medium composition I, a state in which vascular smooth muscle cells are attached to the surface of a carrier, a state in which vascular smooth muscle cells are embedded inside a carrier, a state in which plural vascular smooth muscle cells assemble and form cell aggregations (spheres) and the like. To reduce the risk that cell proliferation and property change occur due to adhesion to a solid phase, vascular smooth muscle cells preferably in a state in which single cells are dispersed in medium composition I, or a state in which plural vascular smooth muscle cells assemble and form cell aggregations (spheres), are subjected to suspension culture (preferably, static suspension culture) in medium composition I. That is, preferably, a carrier for adhesion or embedding of vascular smooth muscle cells is not used. Among these states, the state with forming cell aggregations (spheres) is preferable, since cell-cell interactions and cell structures close to those in the in vivo environment are reconstructed, long-term culture can be performed while maintaining the cell function, and also cell recovery is relatively easy.

A method for forming a cell aggregate (sphere) is not particularly limited, and can be appropriately selected by those of ordinary skill in the art. Examples thereof include a method using a culture container having a cell non-adhesive surface, hanging drop method, gyratory culture method, three-dimensional scaffold method, centrifugation method, a method using coagulation by an electric field or magnetic field and the like.

A sphere can also be formed using the culture composition I. For example, a sphere is prepared by uniformly dispersing the object vascular smooth muscle cells, dissociated to single cells, in culture composition I, and allowing them to culture in suspension by standing still for 3 days to 10 days. The prepared sphere can be collected by performing centrifugation or filtration treatment.

In one embodiment, vascular smooth muscle cells whose cell proliferation is arrested are subjected to culturing. When the culture method of the present invention is used, vascular smooth muscle cells can be maintained for a long term in a state where cell proliferation is arrested while maintaining good viability. The "state where cell proliferation is arrested" refers to a specific proliferation rate (div/day) of the cell of not more than 0.1, preferably not more than 0.001, more preferably not more than 0. In vascular smooth muscle cells with arrested cell proliferation, the cell cycle is generally arrested in $G_0$ or $G_1$ phase. In one embodiment, the cell cycle is arrested in $G_0$ or $G_1$ phase in not less than 70%, preferably not less than 80%, more preferably not less than 90%, further preferably not less than 95%, still more preferably not less than 99%, of the vascular smooth muscle cells to be subjected to culturing. The proportion of the cells arrested in $G_0$ or $G_1$ phase can be determined by staining the cells with 4',6-diamidino-2-phenylindole, and analyzing the intracellular DNA level by flow cytometry (Cell, vol. 87, pages 1069-1078, 1996).

In one embodiment, vascular smooth muscle cells under proliferation are subjected to culture. When vascular smooth muscle cells under proliferation are cultured by the culture method of the present invention, the proliferation is suppressed and cell proliferation is arrested while maintaining good viability. The present invention also provides such method for suppressing cell proliferation of vascular smooth muscle cells. "Under proliferation" means that the specific proliferation rate (div/day) of the cell exceeds 0.1.

When vascular smooth muscle cells are cultivated by the method of the present invention, blood vessel smooth muscle cells prepared separately are added to the culture composition I and mixed to give a uniform dispersion. In this case, the mixing method is not particularly limited and, for example, manual mixing using pipetting and the like, mixing

using instrument such as stirrer, vortex mixer, microplate mixer, shaking machine and the like can be mentioned. After mixing, the obtained suspension of vascular smooth muscle cells may be subjected to static culture, or the culture medium may be rotated, shaken or stirred as necessary. The rotating speed and frequency can be appropriately set according to the object of those of ordinary skill in the art. When the medium composition needs to be exchanged during the static culture period, the vascular smooth muscle cells and the medium composition are separated by centrifugation or filtration treatment, and a fresh medium composition I can be added to the cells. Alternatively, the vascular smooth muscle cells are appropriately concentrated by centrifugation or filtration treatment, and a fresh medium composition mentioned above can be added to the concentrated liquid.

In one embodiment, primary vascular smooth muscle cells separated from the body, or vascular smooth muscle cells separately cultured in vitro (e.g., vascular smooth muscle cells after adhesion culture) are dispersed to single cells or a state close to single cells. The vascular smooth muscle cells are dispersed using an appropriate cell dispersion liquid. Examples of the cell dispersion liquid include EDTA; protease such as trypsin, collagenase IV, metalloprotease and the like, and the like, which can be used singly or in an appropriate combination. The dispersed vascular smooth muscle cells are suspended in the medium composition I, and subjected to suspension culture (preferably, static suspension culture). In the culture, vascular smooth muscle cells are suspended in the state of single cells or sphere in the medium composition I.

The concentration of vascular smooth muscle cells in the culture is not particularly limited as long as the vascular smooth muscle cells can be maintained while suppressing cell proliferation. It is generally $3.0x10^4$ - $3.0x10^6$ cells/ml, preferably $1.0x10^5$ - $1.0x10^6$ cells/ml, more preferably $3.0x10^5$ - $5.0x10^5$ cells/ml.

The culture period of vascular smooth muscle cells in the medium composition I is not particularly limited as long as the vascular smooth muscle cells can be maintained while suppressing cell proliferation. For example, culturing for not less than 12 hr, not less than 24 hr, not less than 48 hr, or not less than 70 hr is possible. Theoretically, the upper limit of the culture period does not exist. However, since excessive culturing of vascular smooth muscle cells for a long term while suppressing cell proliferation may lower the survival rate, the culture period is desirably set to, for example, not more than 30 days, preferably not more than 14 days, more preferably not more than about 211 hr. When the method of the present invention is used, vascular smooth muscle cells can be maintained in vitro for a long term while suppressing cell proliferation.

In one embodiment, vascular smooth muscle cells under proliferation are subjected to suspension culture (preferably, static suspension culture) in the medium composition I until proliferation of the cells is arrested. The time necessary for vascular smooth muscle cells under proliferation to reach cell proliferation arrest is generally not less than 3 hr, preferably not less than 12 hr, more preferably not less than 24 hr.

The temperature for culturing vascular smooth muscle cells is generally 25 to 39°C, preferably 37°C. The $CO_2$ concentration is generally, in culture atmosphere, 4 to 10% by volume, preferably 5% by volume. The oxygen concentration is, in culture atmosphere, 15 - 50% by volume, preferably 20% by volume.

By such culture operation, vascular smooth muscle cells can be maintained in vitro for a long term while suppressing cell proliferation.

(3) Preservation or transport method of vascular smooth muscle cells

The present invention provides a preservation method and a transport method of vascular smooth muscle cells, using the above-mentioned medium composition I. The preservation or transport method of the present invention is characterized in that vascular smooth muscle cells are suspended in the above-mentioned medium composition I. In the preservation or transport method of the present invention, cells and tissues can be preserved or transported in a suspending state (preferably, statically suspending state) under unfrozen conditions, by using the medium composition I.

The medium composition I to be used for preservation or transport may contain, in addition to the composition described in the above-mentioned (1), various components having a cell viability-prolonging effect during preservation of cells in a unfrozen state. Examples of the component include saccharides (excluding polysaccharides) (e.g., monosaccharides, disaccharides), antioxidants (e.g., SOD, vitamin E or glutathione), hydrophilic polymers (e.g., polyvinylpyrrolidone), chelating agents (e.g., EDTA), sugar alcohols (e.g., mannitol, sorbitol), glycerol and the like.

The vascular smooth muscle cells are preferably isolated. In one embodiment, vascular smooth muscle cells in the primary culture are subjected to preservation or transport. In one embodiment, vascular smooth muscle cells whose cell proliferation is arrested are subjected to preservation or transport. In one embodiment, the cell cycle is arrested in $G_0$ or $G_1$ phase in not less than 70%, preferably not less than 80%, further preferably not less than 90%, more preferably not less than 95%, particularly preferably not less than 99%, of the vascular smooth muscle cells subjected to preservation or transport. In one embodiment, vascular smooth muscle cells under proliferation are subjected to preservation or transport. When the method of the present invention is used, since cell proliferation of vascular smooth muscle cells can be suppressed and the state of cell proliferation arrest can be maintained while maintaining high viability under unfrozen conditions, the vascular smooth muscle cells can be preserved or transported for a long term while

avoiding the risk of property change due to freezing and cell proliferation.

In the preservation or transport method of the present invention, vascular smooth muscle cells are dispersed in the medium composition I and placed in a container that can be sealed tightly. Examples of the container include, but are not limited to, flask, plastic bag, Teflon (registered trade mark) bag, tube, culture bag and the like. To avoid leakage of the content, contamination with bacterium from the outside world and the like during preservation or transport, the container housing the dispersion of the vascular smooth muscle cells in the medium composition I is preferably sealed tightly.

While the temperature during preservation or transport is not particularly limited as long as the viability of the vascular smooth muscle cells can be maintained, it is generally not more than 37°C. While a lower temperature can avoid decreasing of the viability of the vascular smooth muscle cells during preservation or transport, preservation or transport is generally performed at a temperature above the melting point of the medium composition I so that the vascular smooth muscle cells will not be frozen. Therefore, the temperature during preservation or transport is generally maintained at -5 to 42°C, preferably 1 - 37°C, more preferably 4 - 32°C, further preferably 18 - 30°C.

To enable preservation or transport of vascular smooth muscle cells in a statically suspending state, the temperature during preservation or transport is preferably a temperature that enables vascular smooth muscle cells to be statically suspended in the medium composition I.

While the period of preservation or transport is not particularly limited as long as vascular smooth muscle cells can be maintained in a viable state in the medium composition I, it is generally not less than 12 hr and not more than 10 days, preferably 1 - 8 days, more preferably 1 - 3 days. During the preservation or transport period, the cells or tissues preferably maintain the state of being statically suspended in the medium composition I.

When the preservation or transport method of the present invention is used, since the vascular smooth muscle cells can be transported under the conditions free of a freezing operation, and it is expected that the property change of vascular smooth muscle cells, which is observed in freezing and proliferation, can be suppressed to the minimum.

Unless particularly indicated, the definition of each term in (3) is the same as that described in the above-mentioned (1) and (2).

[Examples]

The present invention is explained in more detail in the following by concretely describing the Analysis Examples and Experimental Examples of the medium composition to be used in the present invention as Examples; however, the present invention is not limited thereto.

[Experimental Example 1]

Experiment of survival maintenance of vascular smooth muscle cells and NIH3T3 cells

According to the production method described in WO 2014/017513 A1, a medium composition to be used for suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. The obtained aqueous solution of deacylated gellan gum was sterilized at 121°C for 20 min in an autoclave. Using the solution, DMEM/F12 containing deacylated gellan gum at a final concentration of 0.015%(w/v) (manufactured by Kohjin Bio Co., Ltd.) was produced, and 10%(v/v) fetal bovine serum was further added to prepare a medium composition. Successively, rabbit vascular smooth muscle cells (prepared by Laboratory of Pharmaceutical Science, Graduate School and Faculty of Pharmaceutical Sciences, Chiba University) and mouse fibroblast cell line NIH3T3 (manufactured by ATCC) were prepared at $1\times10^5$ cells/mL in the above-mentioned medium composition, and 5 mL was seeded in a 15 ml tube. As a negative control, rabbit vascular smooth muscle cells or NIH3T3 cells were suspended in DMEM free of deacylated gellan gum and dispensed. Successively, this tube was stood still in a $CO_2$ incubator (37°C, 5% $CO_2$) and the cells were cultured in suspension in a static state for a maximum of 211 hr. Trypan Blue reagent (manufactured by Life Technologies) was added to the cultured product after culturing for 12 hr, 24 hr, 36 hr, 48 hr, 70 hr, 115 hr and 211 hr, and viable cell number and total cell number were counted under a microscope. The survival rate was calculated from the total cell number and the viable cell number.

As a result, the survival rate of each cell in the negative control was less than 50% at 12 hr for rabbit vascular smooth muscle cells and at 36 hr for NIH3T3 cells. On the other hand, in the medium containing deacylated gellan gum, the survival rate of the both cells was not less than 50% up to 115 hr. The viable cell number and survival rate of rabbit vascular smooth muscle cells are shown in Table 1 and Table 2, and the viable cell number and survival rate of NIH3T3 cells are shown in Table 3 and Table 4.

[Table 1]

| viable cell number | negative control | deacylated gellan gum 0.015% |
|---|---|---|
| 0 hr | 500000 | 500000 |
| 12 hr | 42000 | 369000 |
| 24 hr | 13000 | 336000 |
| 36 hr | 4000 | 361000 |
| 48 hr | 1000 | 306000 |
| 70 hr | not performed | 304000 |
| 115 hr | not performed | 221000 |
| 211 hr | not performed | 100000 |

[Table 2]

| survival rate (%) | negative control | deacylated gellan gum 0.015% |
|---|---|---|
| 0 hr | 100 | 100 |
| 12 hr | 30.5 | 79.5 |
| 24 hr | 18.6 | 76.6 |
| 36 hr | 9.3 | 72.6 |
| 48 hr | 5.6 | 70.2 |
| 70 hr | not performed | 66.7 |
| 115 hr | not performed | 52.6 |
| 211 hr | not performed | 25.6 |

[Table 3]

| viable cell number | negative control | deacylated gellan gum 0.015% |
|---|---|---|
| 0 hr | 500000 | 500000 |
| 12 hr | 129000 | 474000 |
| 24 hr | 40000 | 489000 |
| 36 hr | 19000 | 488000 |
| 48 hr | 19000 | 389000 |
| 70 hr | not performed | 377000 |
| 115 hr | not performed | 296000 |
| 211 hr | not performed | 140000 |

[Table 4]

| survival rate (%) | negative control | deacylated gellan gum 0.015% |
|---|---|---|
| 0 hr | 100 | 100 |
| 12 hr | 91.3 | 90.0 |
| 24 hr | 74.7 | 90.9 |

(continued)

| survival rate (%) | negative control | deacylated gellan gum 0.015% |
|---|---|---|
| 36 hr | 32.5 | 91. 9 |
| 48 hr | 30.6 | 91.7 |
| 70 hr | not performed | 90.1 |
| 115 hr | not performed | 68.1 |
| 211 hr | not performed | 33.6 |

[Experimental Example 2]

Cell cycle analysis of vascular smooth muscle cells

According to the production method described in WO 2014/017513 A1, a medium composition to be used for suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. The obtained aqueous solution of deacylated gellan gum was sterilized at 121°C for 20 min in an autoclave. Using the solution, DMEM/F12 containing deacylated gellan gum at a final concentration of 0.015%(w/v) (manufactured by Kohjin Bio Co., Ltd.) was produced, and 10%(v/v) fetal bovine serum was further added to prepare a medium composition. Successively, rabbit vascular smooth muscle cells (prepared by Laboratory of Pharmaceutical Science, Graduate School and Faculty of Pharmaceutical Sciences, Chiba University) were prepared at $4\times10^5$ cells/mL in the above-mentioned medium composition, and 9 mL was seeded in a 15 ml tube. Successively, this tube was stood still in a $CO_2$ incubator (37°C, 5% $CO_2$) and the cells were cultured in suspension in a static state for a maximum of 5 days. As a control, rabbit vascular smooth muscle cells were suspended in DMEM (manufactured by Wako Pure Chemical Industries, Ltd.) free of deacylated gellan gum and cultured on a conventional petri dish for monolayer culture.

Protein was recovered from each cell by using cell lysis solution (IP buffer), quantified by the BCA method (Thermo Scientific). Using 20 μg of the protein, Western blot analysis was performed. As primary antibodies (manufactured by Santa Cruz Biotechnology), polyclonal anti-p27 antibody (C-19), polyclonal anti-p21 antibody (M-19), polyclonal anti-CDK2 antibody (M-2), and polyclonal anti-β-actin antibody (I-19) were used at 200-fold dilution. As the secondary antibody, anti-rabbit IgG-HRP antibody (Amersham Bioscience) was used at 10,000-fold dilution. Detection was performed with LAS4000 (FUJIFILM) using ECL Western Blotting Detection Reagents (Amersham Bioscience) or Immobilon Western (Millipore).

Expression of cell cycle factors in the rabbit vascular smooth muscle cells was compared between suspension culture in a deacylated gellan gum-containing medium and monolayer culture in a deacylated gellan gum-free medium. When rabbit vascular smooth muscle cells were cultured in a deacylated gellan gum-containing medium, both the expression of cdk2 that promotes $G_1$/S phase transfer in the cell cycle and the expression of p21 and p27 that suppress $G_1$/S transfer decreased (Fig. 1). This phenomenon was not observed with rabbit vascular smooth muscle cells in the monolayer culture. The results of decrease in both the $G_1$/S phase promoting factor and suppressive factor suggest that the suspension culture in the deacylated gellan gum-containing medium arrested the cell cycle of the rabbit vascular smooth muscle cells at $G_0$ phase.

[Experimental Example 3]

Cell cycle analysis of vascular smooth muscle cells

According to the production method described in WO 2014/017513 A1, a medium composition to be used for suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using the solution, DMEM/F12 at a final concentration of 0.015%(w/v) (manufactured by Kohjin Bio Co., Ltd.) was produced, and 10%(v/v) fetal bovine serum was further added to prepare a medium composition. Successively, rabbit vascular smooth muscle cells (prepared by Laboratory of Pharmaceutical Science, Graduate School and Faculty of Pharmaceutical Sciences, Chiba University) were prepared at $3\times10^5$ cells/mL (rabbit vascular smooth muscle cells) in the above-mentioned medium composition, and 5 mL was seeded in a 15 ml tube. Successively, this tube was stood still in a $CO_2$ incubator (37°C, 5% $CO_2$) and the cells were cultured in suspension in a static state for a maximum of 3 days. As a control, rabbit vascular smooth

muscle cells obtained by suspending in DMEM (manufactured by Wako Pure Chemical Industries, Ltd.) free of deacylated gellan gum and culturing on a conventional petri dish for monolayer culture were used.

Protein was recovered from each cell by using cell lysis solution (IP buffer), quantified by the BCA method (Thermo Scientific). Using 10 - 15 μg of the protein, Western blot analysis was performed. As the primary antibodies, polyclonal anti-cyclin E1 antibody (M-20), polyclonal anti-CDK2 antibody (M-2), polyclonal anti-p27 antibody (C-19), polyclonal anti-p21 antibody (M-19) and polyclonal anti-β-actin antibody (I-19) manufactured by Santa Cruz Biotechnology were used at 200-fold dilution and monoclonal anti-cyclin D1 antibody (HD11) manufactured by Santa Cruz Biotechnology was used at 250-fold dilution, monoclonal anti-cyclin D3 antibody manufactured by BD Biosciences was used at 1000-fold dilution, and monoclonal anti-CDK4 antibody manufactured by BD Biosciences was used at 250-fold dilution. As the secondary antibody, anti-mouse IgG-HRP antibody (GE Healthcare) was used at 50,000-fold dilution, and anti-rabbit IgG-HRP antibody (Cell Signaling Technology) was used on 10,000-fold dilution. Detection was performed with LAS4000 (FUJIFILM) using ECL Western Blotting Detection Reagents (GE Healthcare) or Immobilon Western (Millipore).

Cell cycle factors in the rabbit vascular smooth muscle cells were compared between a deacylated gellan gum-containing medium and monolayer culture. When rabbit vascular smooth muscle cells were cultured in a deacylated gellan gum-containing medium, both the expression of cdk2 that promotes $G_{1/}S$ phase transfer in the cell cycle and the expression of p21 that suppresses $G_{1/}S$ transfer decreased (Fig. 2). This phenomenon was not observed with rabbit vascular smooth muscle cells in the monolayer culture. The results of decrease in both the $G_1/S$ phase promoting factor and suppressive factor suggest that the deacylated gellan gum-containing medium transferred the rabbit vascular smooth muscle cells to the $G_0$ phase.

[Experimental Example 4]

Cell cycle analysis of NIH3T3 cells

According to the production method described in WO 2014/017513 A1, a medium composition to be used for suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using the solution, DMEM/F12 at a final concentration of 0.015%(w/v) (manufactured by Kohjin Bio Co., Ltd.) was produced, and 10%(v/v) fetal bovine serum was further added to prepare a medium composition. Successively, mouse fibroblast cell line NIH3T3 (manufactured by ATCC) was prepared at $3\times10^5$ cells/mL in the above-mentioned medium composition, and 5 mL was seeded in a 15 ml tube. Successively, this tube was cultured in suspension in a static state for maximum 5 days in a $CO_2$ incubator (37°C, 5% $CO_2$). As a control, NIH3T3 cells were used which were obtained by suspending in DMEM (manufactured by Wako Pure Chemical Industries, Ltd.) free of deacylated gellan gum and culturing on a conventional petri dish for monolayer culture.

Protein was recovered from each cell by using cell lysis solution (IP buffer), quantified by the BCA method (Thermo Scientific). Using 10 - 15 μg of the protein, Western blot analysis was performed. As the primary antibodies, polyclonal anti-cyclin E1 antibody (M-20), polyclonal anti-CDK2 antibody (M-2), polyclonal anti-p27 antibody (C-19), polyclonal anti-p21 antibody (M-19) and polyclonal anti-β-actin antibody (I-19) manufactured by Santa Cruz Biotechnology were used at 200-fold dilution and monoclonal anti-cycin D1 antibody (HD11) manufactured by Santa Cruz Biotechnology was used at 250-fold dilution, monoclonal anti-cyclin D3 antibody manufactured by BD Biosciences was used at 1000-fold dilution, and monoclonal anti-CDK4 antibody manufactured by BD Biosciences was used at 250-fold dilution. As the secondary antibodies, anti-mouse IgG-HRP antibody (GE Healthcare) was used at 50,000-fold dilution, and anti-rabbit IgG-HRP antibody (Cell Signaling Technology) was used at 10,000-fold dilution. Detection was performed with LAS4000 (FUJIFILM) using ECL Western Blotting Detection Reagents (GE Healthcare) or Immobilon Western (Millipore).

Cell cycle factors in the NIH3T3 cells were compared between a deacylated gellan gum-containing medium and monolayer culture. When NIH3T3 cells were cultured in a deacylated gellan gum-containing medium, the expression of cdk2 that promotes $G_{1/}S$ phase transfer in the cell cycle decreased, which was similar to the results with rabbit vascular smooth muscle cells. However, the expression of p21 that suppresses $G_1/S$ transfer did not decrease, which was different from the results with rabbit vascular smooth muscle cells (Fig. 3). It was clarified that different cell cycle patterns are obtained by culturing rabbit vascular smooth muscle cells and NIH3T3 cells in a deacylated gellan gum-containing medium.

[Experimental Example 5]

Protein analysis of phosphorylated Erk and phosphorylated FAK in vascular smooth muscle cells

According to the production method described in WO 2014/017513 A1, a medium composition to be used for

suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using the solution, DMEM/F12 at a final concentration of 0.015%(w/v) (manufactured by Kohjin Bio Co., Ltd.) was produced, and 10%(v/v) fetal bovine serum was further added to prepare a medium composition. Successively, rabbit vascular smooth muscle cells (prepared by Laboratory of Pharmaceutical Science, Graduate School and Faculty of Pharmaceutical Sciences, Chiba University) were prepared at $3\times10^5$ cells/mL in the above-mentioned medium composition, and 5 mL was seeded in a 15 ml tube. Successively, this tube was cultured in suspension in a static state for maximum 5 days in a $CO_2$ incubator (37°C, 5% $CO_2$). As a control, rabbit vascular smooth muscle cells obtained by suspending in DMEM (manufactured by Wako Pure Chemical Industries, Ltd.) free of deacylated gellan gum and culturing on a conventional petri dish for monolayer culture were used. Successively, this tube was stood still in a $CO_2$ incubator (37°C, 5% $CO_2$) to culture the cells in suspension in a static state for a maximum of 3 days.

Protein was recovered from each cell by using cell lysis solution (IP buffer), quantified by the BCA method (Thermo Scientific). Using 10 - 15 μg of the protein, Western blot analysis was performed. As the primary antibodies, monoclonal anti-phosphorylated FAK (p125) antibody manufactured by Upstate Biotechnology was used at 100-fold dilution, monoclonal anti-FAK antibody manufactured by BD Biosciences was used at 200-fold dilution, polyclonal anti-phosphorylated p44/42 MAPK (Erk1/2) antibody (Thr202/Tyr204) manufactured by Cell Signaling was used at 500-fold dilution, and monoclonal anti-MAP kinase (ERK1 + ERK2) antibody manufactured by ZYMED was used at 500-fold dilution. As the secondary antibodies, anti-mouse IgG-HRP antibody (GE Healthcare) was used at 50,000-fold dilution, and anti-rabbit IgG-HRP antibody (Cell Signaling Technology) was used at 10,000-fold dilution. Detection was performed by LAS4000 (FUJIFILM) using ECL Western Blotting Detection Reagents (GE Healthcare) or Immobilon Western (Millipore).

Signal transduction factors (Erk and FAK) in rabbit vascular smooth muscle cells were compared between deacylated gellan gum-containing medium and monolayer culture. In the monolayer culture, the expression level of phosphorylated Erk was undetectable in 1 hr from the start of culture. When the rabbit vascular smooth muscle cells were cultured in a medium containing deacylated gellan gum, the expression level of phosphorylated Erk was of the same level as in the monolayer culture. On the other hand, the expression level of phosphorylated FAK was lower than that in the monolayer culture (Fig. 4).

[Experimental Example 6]

Protein analysis of phosphorylated Erk and phosphorylated FAK in NIH3T3 cells

According to the production method described in WO 2014/017513 A1, a medium composition to be used for suspension culture of the cells was prepared. That is, deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using the aqueous solution, DMEM/F12 at a final concentration of 0.015%(w/v) (manufactured by Kohjin Bio Co., Ltd.) was produced, and 10%(v/v) fetal bovine serum was further added to prepare a medium composition. Successively, mouse fibroblast cell line NIH3T3 (manufactured by ATCC) was prepared at $3\times10^5$ cells/mL in the above-mentioned medium composition, and 5 mL was seeded in a 15 ml tube. As a control, NIH3T3 cells obtained by suspending in DMEM (manufactured by Wako Pure Chemical Industries, Ltd.) free of deacylated gellan gum and culturing on a conventional petri dish for monolayer culture were used. Successively, this tube was stood still in a $CO_2$ incubator (37°C, 5% $CO_2$) to culture the cells in suspension in a static state for a maximum of 5 days.

Protein was recovered from each cell by using cell lysis solution (IP buffer), quantified by the BCA method (Thermo Scientific). Using 10 - 15 μg of the protein, Western blot analysis was performed. As the primary antibodies, monoclonal anti-phosphorylated FAK (p125) antibody manufactured by Upstate Biotechnology was used at 100-fold dilution, monoclonal anti-FAK antibody manufactured by BD Biosciences was used at 200-fold dilution, polyclonal anti-phosphorylated p44/42 MAPK (Erk1/2) antibody (Thr202/Tyr204) manufactured by Cell Signaling was used at 500-fold dilution, and monoclonal anti-MAP kinase (ERK1 + ERK2) antibody manufactured by ZYMED was used at 500-fold dilution. As the secondary antibodies, anti-mouse IgG-HRP antibody (GE Healthcare) was used at 50,000-fold dilution, and anti-rabbit IgG-HRP antibody (Cell Signaling Technology) was used at 10,000-fold dilution. Detection was performed by LAS4000 (FUJIFILM) using ECL Western Blotting Detection Reagents (GE Healthcare) or Immobilon Western (Millipore).

Signal transduction factors (Erk and FAK) in NIH3T3 cells were compared between deacylated gellan gum-containing medium and monolayer culture. In the monolayer culture, the expression level of phosphorylated Erk was undetectable. When the cells were cultured in a medium containing deacylated gellan gum, the expression level of phosphorylated Erk was higher than that in the monolayer culture. On the other hand, the expression level of phosphorylated FAK was lower than that in the monolayer culture (Fig. 5). Since the phosphorylation patterns of Erk and FAK are different between the rabbit vascular smooth muscle cells and NIH3T3 cells, the signal transduction was suggested to be different between the both cells.

[Experimental Example 7]

Deacylated gellan gum (KELCOGEL CG-LA, manufactured by SANSHO Co., Ltd.) was suspended in ultrapure water (Milli-Q water) to 0.3%(w/v), and dissolved by stirring with heating at 90°C. This aqueous solution was sterilized at 121°C for 20 min in an autoclave. Using the aqueous solution, DMEM/F12 having a final concentration of 0.015%(w/v) (manufactured by Kohjin Bio Co., Ltd.) was produced, and 10%(v/v) fetal bovine serum was further added to prepare a medium composition. Successively, rabbit vascular smooth muscle cells (prepared by Laboratory of Pharmaceutical Science, Graduate School and Faculty of Pharmaceutical Sciences, Chiba University) were prepared at $1\times10^5$ cells/mL in the above-mentioned medium composition, and seeded in a 15 ml tube. Successively, this tube was cultured by being stood still in a $CO_2$ incubator (37°C, 5% $CO_2$) for 2 days. Thereafter, the cells were treated with trypsin, seeded at $9\times10^4$ cells/3 cm petri dish by using DMEM (manufactured by Wako Pure Chemical Industries, Ltd.), and culturing was continued. The medium was exchanged after 3 days, 5 days and 7 days of culture. The cells after culturing for 1 day, 3 days, 5 days, 7 days and 10 days were recovered, suspended in Trypan Blue reagent (manufactured by Life Technologies), and viable cell number and total cell number were counted under a microscope. The survival rate was calculated from the total cell number and the viable cell number.

Vascular smooth muscle cells were cultured in suspension in a deacylated gellan gum-containing medium while keeping the suspended state, cell proliferation was suppressed while maintaining good viability. While this property was maintained for a given period after passage of vascular smooth muscle cells from suspension culture to adhesion culture on a plate, the vascular smooth muscle cells proliferated thereafter (Fig. 6).

[Industrial Applicability]

According to the present invention, vascular smooth muscle cells can be maintained in vitro for a long term while suppressing cell proliferation. According to the present invention, vascular smooth muscle cells can be transported under conditions free of a freezing operation, and the property change of cells observed when freezing or adhering them is expected to be minimized. The present invention contributes to the development of regenerative medicine using vascular smooth muscle cells.

**Claims**

1. A method for culturing vascular smooth muscle cells, which comprises culturing vascular smooth muscle cells in suspension in a medium composition comprising deacylated gellan gum.

2. The method according to claim 1, wherein the vascular smooth muscle cells in a cell proliferation-arrested state are cultured in suspension.

3. A method for suppressing proliferation of vascular smooth muscle cells, which comprises culturing vascular smooth muscle cells in suspension in a medium composition comprising deacylated gellan gum.

4. A method for preserving vascular smooth muscle cells, which comprises suspending vascular smooth muscle cells in a medium composition comprising deacylated gellan gum.

5. The method according to any one of claims 1 to 4 wherein the medium composition comprises deacylated gellan gum at a concentration of 0.005 - 0.3% (w/v).

**Patentansprüche**

1. Verfahren zum Kultivieren von Zellen der glatten Gefäßmuskulatur, das das Kultivieren von Zellen der glatten Gefäßmuskulatur in Suspension in einer Mediumzusammensetzung, die deacyliertes Gellan umfasst, umfasst.

2. Verfahren gemäß Anspruch 1, wobei die Zellen der glatten Gefäßmuskulatur in einem Zustand der Zellvermehrungsarretierung in Suspension kultiviert werden.

3. Verfahren zum Unterdrücken der Vermehrung von Zellen der glatten Gefäßmuskulatur, das das Kultivieren von Zellen der glatten Gefäßmuskulatur in Suspension in einer Mediumzusammensetzung, die deacyliertes Gellan umfasst, umfasst.

4. Verfahren zum Konservieren von Zellen der glatten Gefäßmuskulatur, das das Suspendieren von Zellen der glatten Gefäßmuskulatur in einer Mediumzusammensetzung, die deacyliertes Gellan umfasst, umfasst.

5. Verfahren gemäß einem der Ansprüche 1 bis 4, wobei die Mediumzusammensetzung deacyliertes Gellan in einer Konzentration von 0,005 bis 0,3% (w/v) umfasst.

**Revendications**

1. Procédé pour cultiver des cellules musculaires lisses vasculaires, qui comprend la culture de cellules musculaires lisses vasculaires en suspension dans une composition de milieu comprenant de la gomme gellane désacylée.

2. Procédé selon la revendication 1, dans lequel les cellules musculaires lisses vasculaires dans un état de prolifération cellulaire bloquée sont cultivées en suspension.

3. Procédé pour supprimer la prolifération de cellules musculaires lisses vasculaires, qui comprend la culture de cellules musculaires lisses vasculaires en suspension dans une composition de milieu comprenant de la gomme gellane désacylée.

4. Procédé pour conserver des cellules musculaires lisses vasculaires, qui comprend la mise en suspension de cellules musculaires lisses vasculaires dans une composition de milieu comprenant de la gomme gellane désacylée.

5. Procédé selon l'une quelconque des revendications 1 à 4, dans lequel la composition de milieu comprend de la gomme gellane désacylée à une concentration de 0,005 à 0,3 % (poids/volume).

Fig. 1

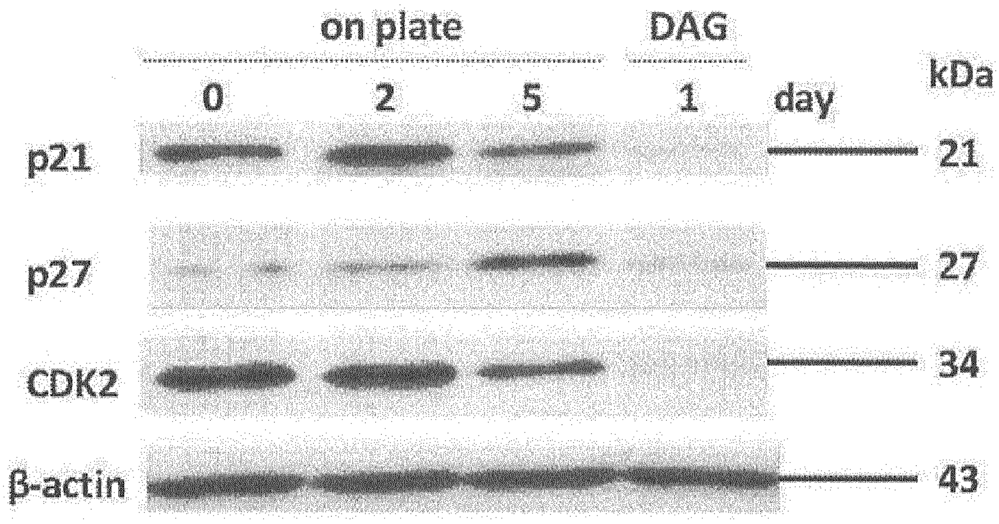
on plate
DAG
0
2
5
1
day
kDa
p21
21
p27
27
CDK2
34
β-actin
43

Fig. 2

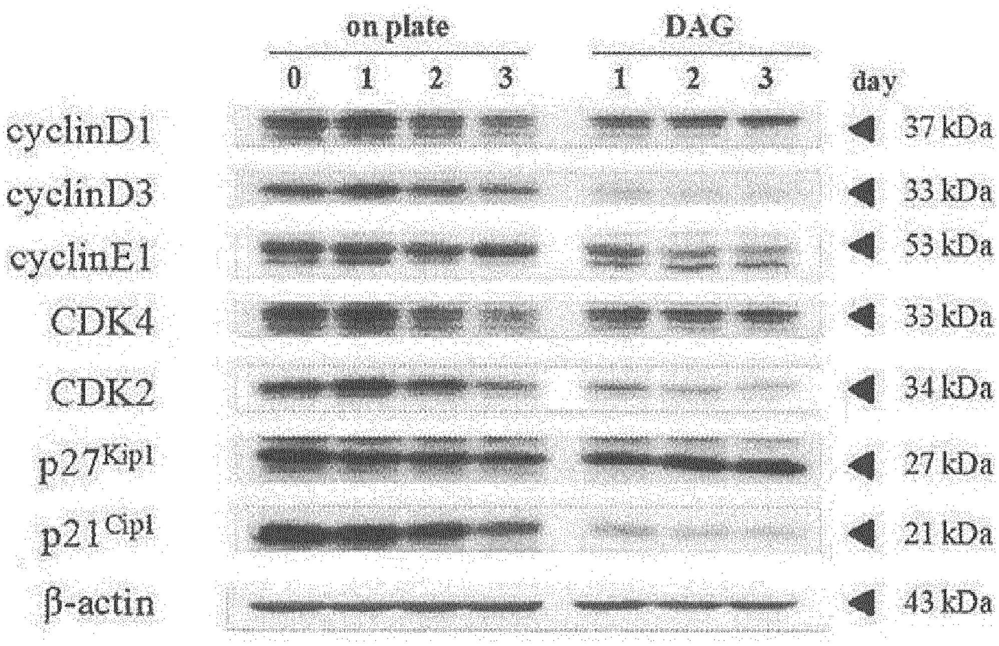
on plate
DAG
0
1
2
3
1
2
3
day
cyclinD1
37 kDa
cyclinD3
33 kDa
cyclinE1
53 kDa
CDK4
33 kDa
CDK2
34 kDa
p27Kip1
27 kDa
p21Cip1
21 kDa
β-actin
43 kDa

Fig. 3

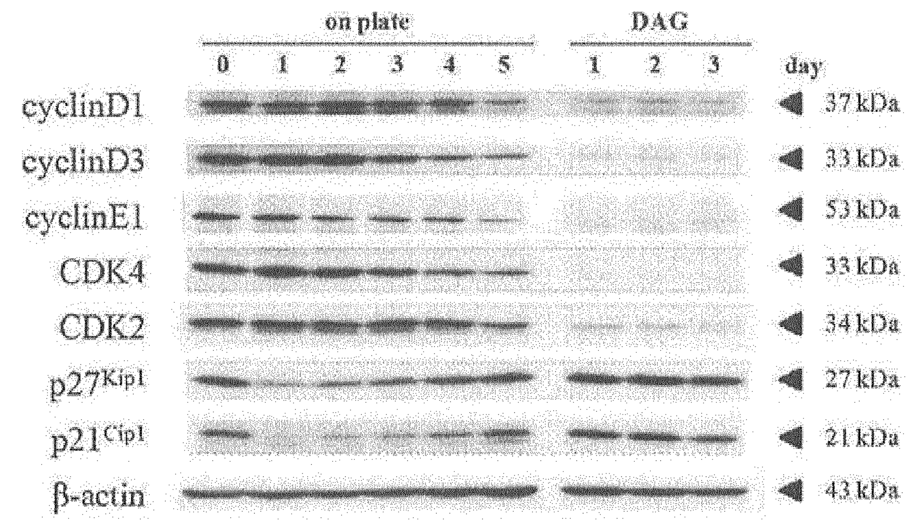
on plate
DAG
0 1 2 3 4 5
1 2 3
day
cyclinD1
cyclinD3
cyclinE1
CDK4
CDK2
p27Kip1
p21Cip1
β-actin
37 kDa
33 kDa
53 kDa
33 kDa
34 kDa
27 kDa
21 kDa
43 kDa

Fig. 4

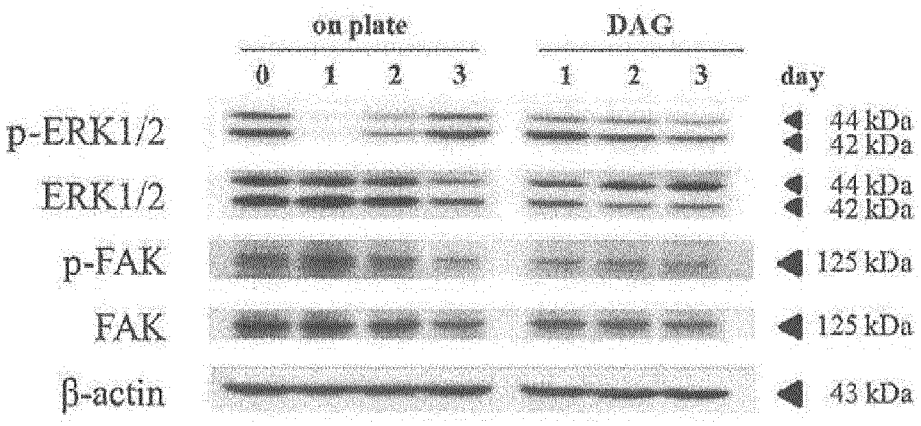
on plate
DAG
0 1 2 3
1 2 3
day
p-ERK1/2
ERK1/2
p-FAK
FAK
β-actin
44 kDa
42 kDa
44 kDa
42 kDa
125 kDa
125 kDa
43 kDa

Fig. 5

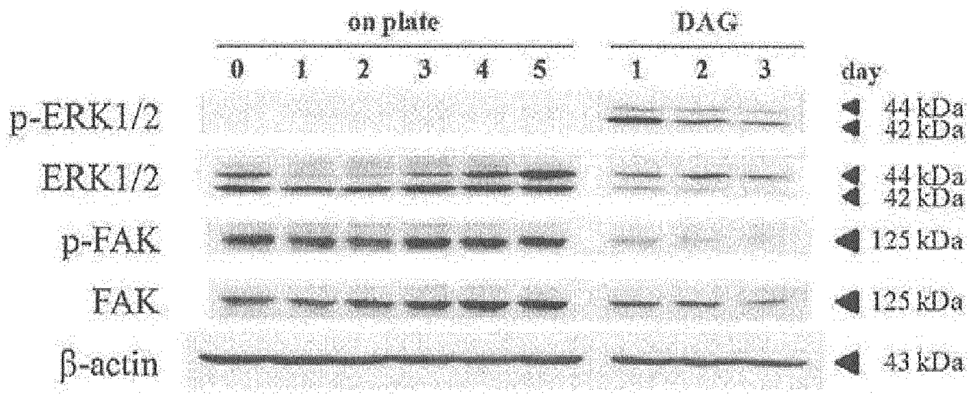
on plate
DAG
0 1 2 3 4 5
1 2 3
day
p-ERK1/2
ERK1/2
p-FAK
FAK
β-actin
44 kDa
42 kDa
44 kDa
42 kDa
125 kDa
125 kDa
43 kDa

Fig. 6

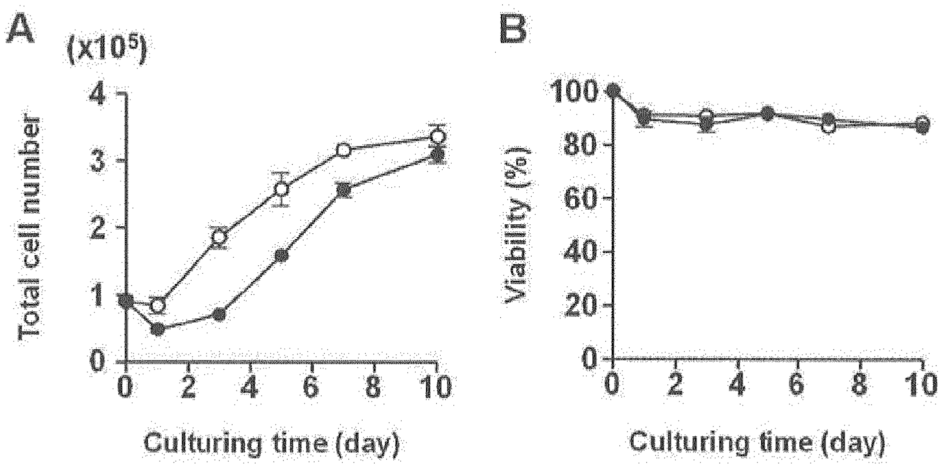
A
(x10^5)
Total cell number
Culturing time (day)
B
Viability (%)
Culturing time (day)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014017513 A1
- US 20140106348 A1
- JP 2004129596 A


### Non-patent literature cited in the description


- **KOYAMA et al.** *Cell,* 1996, vol. 87, 1069-78
- **ISHII et al.** *Atherosclerosis,* 2001, vol. 158, 377-84
- **MAY-JUN et al.** *Arteriosclerosis,* 1984, vol. 4 (3), 183-188
- **OREKHOV et al.** *Med Biol.,* 1984, vol. 62 (4), 255-259
- *Cell,* 1996, vol. 87, 1069-1078
- Laboratory of Pharmaceutical Science, Graduate School and Faculty of Pharmaceutical Sciences. Chiba University